Europäisches Patentamt

European Patent Office

Office européen des brevets

(11) Numéro de publication: **0 430 374 B1**

## FASCICULE DE BREVET EUROPEEN

(45) Date de publication de fascicule du brevet: **21.09.94** (51) Int. Cl.⁵: **A61B 8/06**, G01S 15/58

(21) Numéro de dépôt: **90203123.6**

(22) Date de dépôt: **26.11.90**

(54) **Dispositif de mesure et de visualisation par échographie ultrasonore de paramètres physiologiques d'un écoulement sanguin.**

(30) Priorité: **01.12.89 FR 8915900**

(43) Date de publication de la demande:
**05.06.91 Bulletin 91/23**

(45) Mention de la délivrance du brevet:
**21.09.94 Bulletin 94/38**

(84) Etats contractants désignés:
**DE FR GB**

(56) Documents cités:
**EP-A- 0 225 667**
**DE-A- 3 417 660**

**TOSHIBA REVIEW.WINTER 1986,NO.158, TO-KYO JP pages 20 - 24; Y SEO ET AL:" AN ULTRASOUND BLOOD FLOW IMAGING SYS-TEM,SSH-65A "**

**JAPANESE JOURNAL OF APPLIED PHYSICS, SUPPLEMENTS. vol. 26, no. 1, 1987, TOKYO JA pp.9 - 13; C.KASAI ET AL.: "REAL-TIME TWO-DIMENSIONAL BLOOD FLOWIMAGING USING ULTRASOUND DOPPLER"**

(73) Titulaire: **LABORATOIRES D'ELECTRONIOUE PHILIPS**
**22, avenue Descartes**
**F-94453 Limeil-Brévannes Cédex (FR)**

(84) Etats contractants désignés:
**FR**

(73) Titulaire: **N.V. Philips' Gloeilampenfabrieken**
**Groenewoudseweg 1**
**NL-5621 BA Eindhoven (NL)**

(84) Etats contractants désignés:
**DE GB**

(72) Inventeur: **Bonnefous, Odile**
**Sociéte Civile S.P.I.D.,**
**156 BD Haussmann**
**F-75008 Paris (FR)**

(74) Mandataire: **Pyronnet, Jacques et al**
**Société Civile S.P.I.D.**
**156, Boulevard Haussmann**
**F-75008 Paris (FR)**

## Description

La présente invention concerne un dispositif de mesure et de visualisation de paramètres physiologiques d'un écoulement sanguin, comprenant une unité de mesure par échographie ultrasonore de la vitesse $V(t,z)$ dudit écoulement en fonction du temps $t$ et de la profondeur $z$ d'exploration.

L'invention trouve une application particulièrement avantageuse dans le domaine général de l'exploration échographique d'écoulements sanguins dans les vaisseaux, et plus particulièrement, dans celui de la mesure et de la visualisation, aux fins de diagnostic, de paramètres physiologiques caractéristiques desdits écoulements.

L'étude clinique par échographie ultrasonore des écoulements sanguins est réalisée actuellement avec des appareils mettant en oeuvre l'effet Doppler qui rend compte de la différence de fréquence, appelée fréquence Doppler $f_D$ entre l'onde émise par un transducteur piézoélectrique et l'onde reçue après intéraction avec l'écoulement considéré. La vitesse V du flux sanguin est liée à $f_D$ par la relation :

$$f_D = 2(V/C)f_E \, \mathrm{Cos}\theta \qquad (1)$$

$f_E$ étant la fréquence de l'onde ultrasonore émise, $\theta$ l'angle entre le faisceau ultrasonore et la direction de l'écoulement, et C la vitesse de propagation du son.

Les dispositifs Doppler connus permettent, notamment, d'accéder à des paramètres intéressant particulièrement les praticiens comme le spectogramme des vitesses donnant la distribution de la vitesse d'écoulement V en fonction du temps t. Toutefois, le résultat obtenu est entaché d'une certaine imprécision liée de façon inhérente au principe même de la méthode et qui provient du fait que la fréquence $f_E$ émise par le transducteur piézoélectrique présente une certaine distribution qui se répercute sur la vitesse V via la relation (1) rappelée ci-dessus.

Aussi, le problème technique à résoudre par l'objet de la présente invention est de réaliser un dispositif de mesure et de visualisation de paramètres physiologiques d'un écoulement sanguin, conforme au préambule, qui permettrait d'obtenir un spectogramme des vitesses plus précis et qui conduirait à la détermination de paramètres physiologiques supplémentaires.

La solution au problème technique posé consiste, selon la présente invention, en ce que, la mesure de la vitesse $V(t,z)$ étant indépendante de la fréquence de l'onde ultrasonore utilisée, ledit dispositif de mesure et de visualisation comprend, en sortie d'une mémoire de stockage des valeurs de la vitesse $V(t,z)$, d'une part, un multiplexeur qui, pour un instant donné t, inscrit dans un registre i d'une mémoire le nombre $N_i(t)$ d'échantillons de mesure présentant la valeur $V_i$ à $\Delta V$ près, $\Delta V$ étant le pas de discrétisation, et, d'autre part, un dispositif d'affichage de la vitesse en fonction du temps, tel que la brillance au point $(V_i,t)$ soit proportionnelle à $N_i(t)$.

Parmi les dispositifs pouvant donner une mesure de vitesses indépendantes de la fréquence ultrasonore, on peut citer ceux qui fonctionnent sur le principe de la corrélation temporelle déjà décrite dans la demande de brevet européen n° 0 225 667, et dont l'unité de mesure de la vitesse d'écoulement comprend un circuit d'intercorrélation délivrant à partir de deux échos successifs des valeurs de fonction de corrélation, et un circuit de multiplexage-interpolation fournissant à partir desdites valeurs des fonctions de corrélation une estimée de la vitesse $V(t,z)$.

La précision obtenue sur la mesure de la vitesse d'écoulement permet d'envisager, outre la visualisation du flux sanguin en mode M, par exemple, et la représentation du spectogramme des vitesses, de calculer d'autres paramètres physiologiques jusque là inaccessibles avec les systèmes Doppler conventionnels du fait de leur mauvaise résolution en profondeur. Parmi les paramètres dont la présente invention autorise la détermination figurent le diamètre d'écoulement $D(t)$, la vitesse moyenne $\hat{V}(t)$ et le débit $Q(t)$ du flux sanguin. Les moyens techniques de calcul de ces divers paramètres seront précisés en détail dans la description qui va suivre, en regard des dessins annexés, donnés à titre d'exemples non limitatifs.

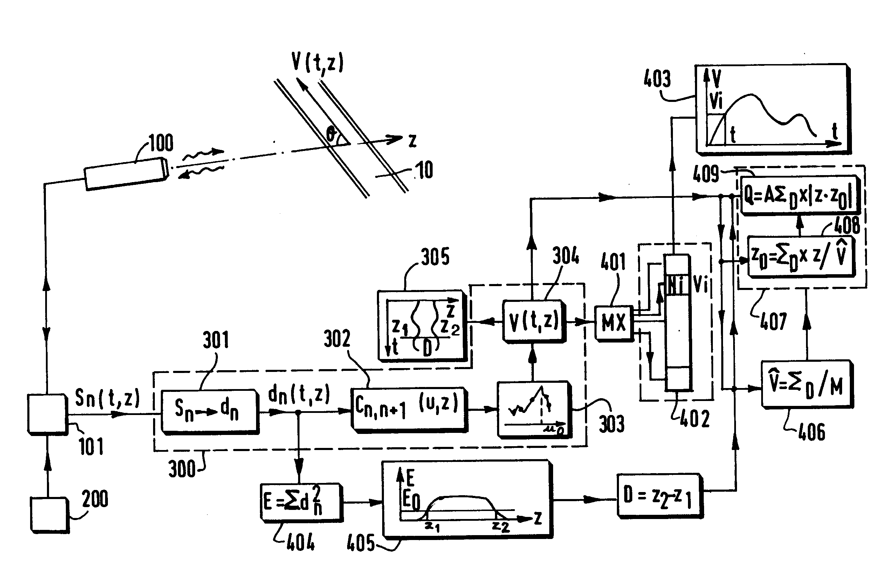

La figure 1 est le schéma d'un dispositif de mesure et de visualisation selon l'invention.

Le schéma de la figure 1 montre un dispositif de mesure et de visualisation de paramètres physiologiques d'un écoulement sanguin 10. Ce dispositif comprend un transducteur piézoélectrique 100 qui peut être, par exemple, une barrette multiéléments. Un étage 200 d'émission relié au transducteur 100 assure la formation d'un faisceau ultrasonore d'exploration tandis qu'une unité 300 de mesure traite les signaux échographiques renvoyés vers le transducteur 100 de façon à fournir une estimée de la vitesse $V(t,z)$ de l'écoulement en fonction du temps t et de la profondeur z d'exploration.

De façon classique, l'étage d'émission 200 comprend un séquenceur composé d'un oscillateur et d'un diviseur de fréquence qui commande à la fréquence de récurrence $1/T$ choisie un générateur dont les signaux électriques d'excitation sont envoyés vers le transducteur 100 qui les convertit en trains périodiques de signaux impulsionnels ultrasonores. Un séparateur 101 des étages d'émission 200 et de mesure 300 est inséré entre le transducteur 100 et lesdits étages 200, 300 et évite l'aveuglement des circuits de mesure par les signaux d'émission.

L'unité 300 de mesure de la vitesse V(t,z) montrée à la figure 1 comporte un éliminateur 301 d'échos fixes, à deux points par exemple, qui fournit du signal reçu $S_n(t,z)$ un signal $d_n(t,z)$ débarassé de ses composantes fixes dues aux réflexions spéculaires sur les parois du vaisseau sanguin examiné. Le signal $d_n(t,z)$ issu de l'éliminateur 301 d'échos fixes est ensuite traité selon la méthode dite d'intercorrélation décrite dans la demande de brevet européen n° 0 225 667 et qui utilise le fait que les signaux ultrasonores rétrodiffusés par une cible en mouvement sont reliés par l'équation suivante :

$$d_{n+1}(t) = d_n(t-\tau)$$

ceci signifie que le signal $n+1$ est la réplique du signal n précédent à un décalage temporel $\tau$ près. Ce dernier représente le temps supplémentaire nécessaire à l'onde ultrasonore pour parcourir le trajet transducteur-cible-transducteur, d'un tir à l'autre. Autrement dit :

$$\tau = 2VT/C$$

où V est la vitesse de la cible et C la vitesse du son. On voit qu'une mesure de $\tau$ permet de mesurer la vitesse V cherchée.

La fonction d'intercorrélation entre $d_n(t)$ et $d_{n+1}(t)$ définie par :

$$C_{n,n+1}(to,u) = \int_{to}^{to+W} d_{n+1}(t+u)d_n(t)dt$$

vérifie :

$$C_{n,n+1}(to,u) = C_{n,n}(to,u-\tau)$$

Le temps to est lié à la profondeur d'exploration z par $to = 2z/C$, et W est la largeur de la fenêtre d'intégration.

La fonction $C_{nn}(to,u)$ est une fonction d'autocorrélation, et, de ce fait, est maximale pour $u=o$. Ainsi, une mesure du décalage temporel $\tau$, et donc de la vitesse V, peut se faire en cherchant pour quel paramètre u la fonction $C_{n,n+1}(to,u)$ est maximale. A cet effet, la fonction d'intercorrélation est échantillonnée, à un pas d'échantillonnage $\Delta t$, entre $u_{min} = -I\Delta t$ et $u_{max} = I\Delta t$ par pas de 1 de façon à obtenir $2I+1$ valeurs de fonctions de corrélation. La valeur maximum de ces $2I+1$ valeurs correspondant à $u=uo$ permet de mesurer $\tau$ en utilisant l'égalité $\tau = uo$. Le calcul des fonctions de corrélation est réalisé par le circuit 302 d'intercorrélation montré à la figure 1.

Afin de s'affranchir des erreurs inhérentes à l'échantillonnage dans la détermination du maximum de la fonction de corrélation, il est possible d'utiliser un circuit 303 de multiplexage-interpolation fournissant à partir des valeurs des fonctions de corrélation une estimée plus précise de la vitesse et la valeur du pic de corrélation correspondant. La demande de brevet européen n° 0 225 667 donne un exemple de ce type de traitement du signal échographique dans lequel la corrélation entre signaux est une corrélation dite "1 bit" en ce sens que les signaux $d_{n+1}$ et $d_n$ utilisés précédemment sont réduits au signe du signal ultrasonore. On sait que dans ce cas, le pic de la fonction de corrélation est de forme triangulaire isocèle. La connaissance de cette forme permet à partir du point le plus haut et de ses deux voisins de reconstituer complètement, par interpolation linéaire, le pic de corrélation et donc de déterminer avec précision l'emplacement de uo.

Contrairement aux vélocimètres Doppler traditionnels, la vitesse d'écoulement V(t,z) ainsi déterminée présente l'avantage d'être insensibles à la dispersion de la fréquence de l'onde ultrasonore utilisée, ce qui permet d'envisager une exploitation beaucoup plus complète des résultats.

Les valeurs trouvées pour la vitesse V(t,z) sont stockées dans une mémoire 304 de stockage en vue de traitements ultérieurs. En particulier, à l'aide de moyens de visualisation connus, il est possible d'afficher sur un premier écran 305 une image de l'écoulement étudié selon la présentation, dite en "mode M", de l'évolution au cours du temps du profil de vitesse dans le vaisseau. Le codage de la vitesse est celle utilisée dans les sytèmes CFM (Color Flow Mapping) : par exemple, le rouge code un sens de

EP 0 430 374 B1

déplacement, le bleu le sens inverse et l'intensité de la vitesse est codée par l'intensité de la couleur.

Un autre traitement particulièrement important pour le médecin est la réalisation d'un spectogramme de vitesse précis. A cet effet, le dispositif de mesure et de visualisation de la figure 1 comprend, en sortie de la mémoire 304 de stockage, d'une part, un multiplexeur 401 qui, pour un instant t donné, inscrit dans un registre i d'une mémoire 402 le nombre Ni(t) d'échantillons de mesure présentant la valeur Vi à $\Delta V$ près, $\Delta V$ étant le pas de discrétisation, et, d'autre part, un dispositif 403 d'affichage de la vitesse en fonction du temps tel que la brillance au point (Vi,t) est proportionnelle à Ni(t).

La figure 1 montre également comment peuvent être calculés d'autres paramètres physiologiques caractéristiques de l'écoulement. Il s'agit d'abord du diamètre D(t) de l'écoulement dont la mesure met en oeuvre, en sortie de l'éliminateur 301 d'échos fixes, un circuit 404 de calcul de l'énergie locale

$$E(t,z) = \sum_n d^2_n(t,z)$$

et un circuit 405 de calcul du diamètre d'écoulement D(t) = $z_2(t)$-$z_1(t)$ constitué par un détecteur de seuil de valeur Eo ajustable, $z_1(t)$ et $z_2(t)$ étant définis par $E(t,z_1(t)) = E(t,z_2(t)) = Eo$.

Connaissant, à chaque instant t, le diamètre D(t) de l'écoulement, il est possible de calculer la vitesse moyenne $\hat{V}(t)$ du flux sanguin grâce à un circuit 406 constitué par un additionneur donnant $\Sigma_D V(t,z)$ et par un diviseur par M, M étant le nombre d'échantillons de la vitesse V(t,z) utilisés sur le segment $[z_1,z_2]$.

De même, le débit Q(t) peut être mesuré par un circuit 407 comprenant, d'une part, un circuit 408 de calcul du centre de gravité $z_o(t) = \Sigma_D V(t,z)z/\hat{V}(t)$, constitué d'un additionneur donnant $\Sigma_D V(t,z)z$ et d'un diviseur par la vitesse moyenne $\hat{V}(t)$ calculée par le circuit 406, et, d'autre part, un additionneur 401 donnant $\Sigma_D V(t,z)|z-z_o|$ suivi d'un multiplieur par la constante A = $\pi\cos\theta/\sin^2\theta$, $\theta$ étant l'angle de l'écoulement avec le faisceau ultrasonore.

## Revendications

**1.** Dispositif de mesure et de visualisation de paramètres physiologiques d'un écoulement sanguin (10), comprenant une unité de mesure (300) par échographie ultrasonore de la vitesse V(t,z) dudit écoulement en fonction du temps t et de la profondeur z d'exploration, caractérisé en ce que, la mesure de la vitesse V(t,z) étant indépendante de la fréquence de l'onde ultrasonore utilisée, ledit dispositif de mesure et de visualisation comprend, en sortie d'une mémoire (304) de stockage des valeurs de la vitesse V(t,z), d'une part, un multiplexeur (401) qui, pour un instant t donné, inscrit dans un registre i d'une mémoire (402) le nombre Ni(t) d'échantillons de mesure présentant la valeur Vi à $\Delta V$ près, $\Delta V$ étant le pas de discrétisation, et, d'autre part, un dispositif (403) d'affichage de la vitesse en fonction du temps tel que la brillance au point (Vi,t) est proportionnelle à Ni(t).

**2.** Dispositif de mesure et de visualisation selon la revendication 1, caractérisé en ce que, comprenant un circuit (404) de calcul de l'énergie locale E(t,z), il comporte également un circuit (405) de calcul du diamètre d'écoulement D(t) = $z_2(t)$-$z_1(t)$ constitué par un détecteur de seuil de valeur Eo, $z_2(t)$ et $z_1(t)$ étant définis par $E(t,z_1(t)) = E(t,z_2(t)) = Eo$.

**3.** Dispositif de mesure et de visualisation selon la revendication 2, caractérisé en ce qu'il comporte un circuit (406) de calcul de la vitesse moyenne $\hat{V}(t)$ constitué par un additionneur donnant $\Sigma_D V(t,z)$ et par un diviseur par M, M étant le nombre d'échantillons de mesure sur le segment $[z_1,z_2]$.

**4.** Dispositif de mesure et de visualisation selon la revendication 3, caractérisé en ce qu'il comporte un circuit (407) de calcul du débit Q(t) comprenant, d'une part, un circuit (408) de calcul du centre de gravité zo(t) de l'écoulement, constitué d'un additionneur donnant $\Sigma_D V(t,z)z$ et d'un diviseur par la vitesse moyenne $\hat{V}(t)$, et, d'autre part, un additionneur (409) donnant $\Sigma_D V(t,z)|z-zo|$ suivi d'un multiplieur par la constante A = $\pi\cos^2/\sin^2\theta$, $\theta$ étant l'angle de l'écoulement avec le faisceau ultrasonore.

**5.** Dispositif de mesure et de visualisation selon l'une quelconque des revendications 1 à 4, caractérisé en ce que l'unité de mesure de la vitesse d'écoulement comprend un circuit (302) d'intercorrélation délivrant, à partir de deux échos successifs, des valeurs de fonctions de corrélation, et un circuit (303) de multiplexage-interpolation fournissant à partir desdites valeurs de corrélation une estimée de la vitesse V(t,z).

4

**Claims**

1. A device for measurement and display of physiological parameters of a blood flow (10), comprising a unit (300) for measuring, using ultrasonic echography, the speed $V(t,z)$ of said flow as a function of the time t and the scanning depth z, characterized in that, the measurement of the speed $V(t,z)$ being independent of the frequency of the ultrasonic wave used, said device for measurement and display comprises, connected to an output of a memory (304) for storing values of the speed $V(t,z)$, on the one hand a multiplexer (401) which writes, for a given instant t, the number $N_i(t)$ of measurement samples having the value $V_i$ to within $\Delta V$, $\Delta V$ being the discretization step, into a register i of a memory (402), and on the other hand a device (403) for displaying the speed as a function of time such that the brightness at the point $(V_i,t)$ is proportional to $N_i(t)$.

2. A device for measurement and display as claimed in Claim 1, characterized in that it comprises a circuit (404) for calculating the local energy $E(t,z)$ and also a circuit (405) for calculating the flow diameter $D(t) = z_2(t) - z_1(t)$ which is formed by a threshold detector for the value Eo, $z_2(t)$ and $z_1(t)$ being defined by $E(t,z_1(t)) = E(t,z_2(t)) = Eo$.

3. A device for measurement and display as claimed in Claim 2, characterized in that it comprises a circuit (406) for calculating the mean speed $\widehat{V}(t)$, which is formed by an adder which supplies $\Sigma_D V(t,z)$ and a divider-by-M, M being the number of measurement samples in the segment $[z_1, z_2]$.

4. A device for measurement and display as claimed in Claim 3, characterized in that it comprises a circuit (407) for calculating the flow rate $Q(t)$, comprising on the one hand a circuit (408) for calculating the centre of gravity $zo(t)$ of the flow, formed by an adder which supplies $\Sigma_D V(t,z)z$ and a divider-by-the-mean-speed $\widehat{V}(t)$, and on the other hand an adder (409) which supplies $\Sigma_D V(t,z)|z-zo|$, followed by a multiplier by the constant $A = \pi\cos^2/\sin^2\theta$, $\theta$ being the angle of the flow relative to the ultrasonic beam.

5. A device for measurement and display as claimed in any one of the Claims 1 to 4, characterized in that the unit for measuring the flow speed comprises an intercorrelation circuit (302) which supplies correlation function values on the basis of two successive echoes, and a circuit (303) for multiplexing/interpolation which supplies an estimate of the speed $V(t,z)$ on the basis of said correlation values.

**Patentansprüche**

1. Anordnung zum Messen und Darstellung physiologischer Parameter einer Blutströmung (10), mit einer Einheit (300) zum Messen der Geschwindigkeit $V(t,z)$ der Strömung abhängig von der Zeit t und der Abtasttiefe z unter Verwendung von Ultraschallechographie, dadurch gekennzeichnet, daß die Messung der Geschwindigkeit $V(t,z)$ von der Frequenz der benutzten Ultraschallwelle unabhängig ist, die Anordnung zum Messen und Darstellen in Verbindung mit einem Ausgang eines Speichers (304) zum Speichern der Werte der Geschwindigkeit $V(t,z)$ einerseits einen Multiplexer (401), der zu einem vorgegebenen Zeitpunkt t die Anzahl $N_i(t)$ der Meßproben mit dem Wert $V_i$ bis zu $\Delta V$ einschreibt, worin $\Delta V$ der Diskretisierungsschritt ist, in ein Register i eines Speichers (402) und andererseits eine Anordnung (403) zum Darstellen der Geschwindigkeit in zeitlicher Abhängigkeit derart enthält, daß die Helligkeit am Punkt $(V_i,t)$ proportional $N_i(t)$ ist.

2. Anordnung zum Messen und Darstellen nach Anspruch 1, dadurch gekennzeichnet, daß sie neben einer Schaltung (404) zum Berechnen der örtlichen Energie $E(t,z)$ ebenfalls eine Schaltung (405) zum Berechnen des Strömungsdurchmessers $D(t) = z_2(t) - z_1(t)$ enthält, die aus einem Schwellenwertdetektor Eo besteht, worin $z_2(t)$ und $z_1(t)$ durch $E(t,z_1(t)) = E(t,z_2(t)) = Eo$ definiert ist.

3. Anordnung zum Messen und Darstellen nach Anspruch 2, dadurch gekennzeichnet, daß sie eine Schaltung (406) zum Berechnen der mittleren Geschwindigkeit $\widehat{V}(t)$ enthält, die aus einem Addierer, der $\Sigma_D V(t,z)$ liefert, und aus einem Teiler durch M besteht, worin M die Anzahl der Meßabtastungen am Segment $[z_1, z_2]$ ist.

4. Anordnung zum Messen und Darstellen nach Anspruch 3, dadurch gekennzeichnet, daß sie eine Schaltung (407) zum Berechnen der Durchflußmenge $Q(t)$ enthält, die einerseits eine Schaltung (408) zum Berechnen der Schwerkraftmenge $zo(t)$ der Strömung, die aus einem Addierer, der $\Sigma_D V(t,z)z$

liefert, und aus einem Teiler durch die mittlere Geschwindigkeit $\hat{V}(t)$ besteht, und andererseits einen Addierer (409), der $\Sigma_D V(t,z)|z\text{-}z_0|$ liefert, gefolgt von einem Vervielfacher mit der Konstante A $=\pi\cos^2/\sin^2\theta$, worin $\theta$ der Strömungswinkel mit dem Ultraschallbündel ist.

5. Anordnung zum Messen und Darstellen nach Anspruch 1 bis 4, <u>dadurch gekennzeichnet</u>, daß die Einheit zum Messen der Strömungsgeschwindigkeit eine Zwischenkorrelationsschaltung (302), die, ausgehend von zwei aufeinanderfolgenden Echos Korrelationsfunktionswerte ausgibt, und eine Multiplex- und Interpolationsschaltung (303) enthält, die, ausgehend von den Korrelationswerten, eine Schätzung der Geschwindigkeit V(t,z) gibt.